# FASCICULE DE BREVET EUROPEEN

(11) **EP 0 624 107 B1**
(45) Date de publication et mention de la délivrance du brevet: **06.09.1995**
(21) Numéro de dépôt: 93904139.8
(22) Date de dépôt: 29.01.1993
(51) Int. Cl.: A61N 1/30, H03K 17/94

(54) **DISPOSITIF DE COMMANDE A DISTANCE DE L'INTENSITE DELIVREE PAR UN GENERATEUR DE COURANT AVEC UNE MAIN PLONGEANT DANS UN LIQUIDE CONDUCTEUR**
FERNSTEUERUNGSEINRICHTUNG FÜR DIE VON EINEM STROMGENERATOR GELIEFERTE STROMSTÄRKE MIT EINER IN EINE LEITFÄHIGE FLÜSSIGKEIT EINTAUCHENDEN HAND
DEVICE FOR REMOTELY CONTROLLING THE OUTPUT OF A CURRENT GENERATOR WITH ONE HAND IMMERSED IN A CONDUCTIVE LIQUID

(30) Priorité: 30.01.1992 FR 9201014
(43) Date de publication de la demande: 17.11.1994
(73) Titulaire: I2M-INNOVATION MATERIEL MEDICAL, F-14000 Caen (FR)
(72) Inventeur: DESNOS, Philippe, F-14000 Caen (FR)
(74) Mandataire: Cabinet HERRBURGER
(86) Numéro de dépôt international: FR9300097
(87) Numéro de publication internationale: WO9314812

(56) Documents cités:
- DE-U- 8 620 271
- DE-U- 8 715 456
- DE-U- 8 813 498
- DE-U- 9 007 277
- US-A- 4 329 581

## Description

La présente invention se rapporte à un dispositif permettant à un utilisateur de commander à distance l'intensité délivrée par un générateur de courant relié à deux électrodes plongeant, respectivement, dans deux bacs contenant un liquide conducteur de l'électricité, tel que de l'eau ou une solution aqueuse, avec l'une de ses mains plongeant dans ce liquide.

Un tel dispositif est spécialement adapté au traitement des hyperhidroses palmoplantaires par ionophorèse. Un exemple d'un tel dispositif est donné dans le document DE-U-8 715 456.

La transpiration est un phénomène physiologique qui est normal et nécessaire pour permettre à l'organisme ce lutter contre la chaleur, et le cas échéant, d'éliminer des substances toxiques. Il arrive, cependant parfois, que chez certains sujets celle-ci devienne excessive au point de les gêner dans leur vie quotidienne et ce correspondre à une véritable invalidité, notamment lorsqu'elle se produit au niveau des mains ou des pieds : c'est l'hyperhidrose palmoplantaire.

Compte tenu du caractère particulièrement gênant de cette affection, les spécialistes ont cherché à proposer divers traitements tant chirurgicaux que médicamenteux pour lutter contre celle-ci ; la plupart de ces traitements se sont, malheureusement, avérés très décevants.

En fait, le seul traitement qui a, jusqu'à présent, permis de donner des résultats positifs, sans être trop contraignant est l'ionophorèse, qui s'est développée en France depuis 1986, et qui consiste à faire circuler un courant galvanique d'environ 20 mA dans les régions à traiter.

Ce traitement permet, en effet, d'obtenir d'excellents résultats tant à court terme qu'à long terme, dans la mesure où le protocole d'attaque est respecté et où des séances d'entretien sont correctement effectuées.

On peut estimer, actuellement, le nombre de personnes ayant bénéficié de ce traitement à un chiffre compris entre 10 et 20 000 pour la France.

Dans le cas particulier de l'hyperhidrose palmoplantaire, c'est-à-dire du traitement des mains et des pieds, on utilise un appareillage constitué par deux bacs contenant un liquide conducteur de l'électricité tel que de l'eau ou une solution aqueuse, et dans lesquels plonge respectivement une électrode en acier reliée à un générateur de courant : l'utilisateur met chaque main ou chaque pied ou une main et un pied dans l'un de ces deux bacs pour permettre la circulation du courant traitant.

Pour garantir le confort de l'utilisateur, il est impératif qu'au début du traitement, le courant soit augmenté de façon progressive, et, qu'à la fin de celui-ci, le courant diminue également de manière progressive ; en effet, si l'intensité du courant varie trop rapidement ou à l'extrême est établie ou coupée brutalement, l'utilisateur reçoit une forte décharge électrique qui le dissuade de continuer le traitement.

Cette situation fait que, lorsque l'on veut traiter simultanément les deux mains, la présence d'un tiers est obligatoire pour régler l'appareil. Il en est, bien entendu, de même si l'on veut traiter simultanément les deux mains et les deux pieds ; ce n'est que dans le cadre d'un traitement n'intéressant simultanément qu'une main et un pied que l'utilisateur peut régler lui même l'appareillage.

On n'a pas, jusqu'à présent, trouvé de moyen pour permettre de remédier à cet inconvénient dont la conséquence est de multiplier par deux le temps de traitement, ce qui est particulièrement fâcheux.

La présente invention a pour objet de combler cette lacune en proposant un dispositif permettant à un utilisateur de commander, à distance, l'appareillage susmentionné, directement avec l'une de ses mains plongeant dans le liquide des bacs de traitement, sans avoir à sortir celle-ci, et ce, en garantissant sa sécurité ainsi que la bonne marche du processus de traitement par ionophorèse.

Selon l'invention, ce dispositif est caractérisé en ce qu'il comporte au moins une fibre optique conduisant la lumière ambiante et dont une extrémité ou première extrémité est susceptible d'être obturée manuellement par l'utilisateur, de façon à commander l'augmentation ou la diminution du courant délivré par le générateur.

L'utilisation d'une telle fibre optique, ou le cas échéant d'un faisceau de fibres optiques, permet, en effet, de manière surprenante, de résoudre tous les problèmes tant de réalisation, de sécurité, de fiabilité que de faisabilité qui sont inhérents au réglage à distance dans l'eau d'un générateur de courant.

Conformément à la configuration préférentielle de l'invention, il est prévu, au moins, deux fibres optiques, et l'obturation manuelle de l'une de ces fibres commande l'augmentation de l'intensité du courant délivré par l'appareillage, tandis que l'obturation de l'autre fibre commande la diminution de l'intensité de ce courant.

Le dispositif conforme à l'invention permet ainsi à un utilisateur de traiter lui-même sans l'intervention d'un tiers, ses deux mains, et même ses deux mains et ses deux pieds, si l'appareil d'ionophorèse dispose de deux générateurs.

Le réglage de cet appareil est extrêmement simple étant donné que la seule manoeuvre à effectuer consiste à obturer, avec le doigt, l'extrémité d'une fibre optique.

Selon une autre caractéristique de l'invention, chacune des fibres optiques coopère, par sa seconde extrémité, opposée à la première extrémité pouvant être obturée par l'utilisateur, avec un commutateur opto-électronique respectif susceptible de commander la fermeture d'un circuit de réglage respectif renfermant un générateur de courant annexe ainsi qu'un organe de commande central.

Conformement à la configuration préférentielle de l'invention, qui correspond à un dispositif muni de deux fibres optiques commandant respectivement l'augmentation et la diminution de l'intensité du courant de traitement, il est prévu deux commutateurs électroniques associés, chacun, à un circuit de réglage, et susceptibles d'actionner ce circuit.

Selon une autre caractéristique de l'invention, chacun des commutateur opto-électroniques comporte un phototransistor dont la base coopère avec la seconde extrémité de la fibre optique associée, un amplificateur susceptible d'amplifier fortement le signal transmis par le phototransistor, ainsi qu'un connecteur dont l'actionnement par ce signal permet de commander la fermeture du circuit de réglage associé.

Compte tenu de cette configuration, les commutateurs opto-électroniques peuvent être comparés à des relais susceptibles de basculer une porte logique de façon à relier l'organe de commande central à un générateur de courant.

Selon une autre caractéristique de l'invention, l'organe de commande central comporte un condensateur chargé en tension associé à un amplificateur opérationnel, et coopérant avec un convertisseur tension/courant.

Les caractéristiques du dispositif susmentionné seront décrites plus en détail en se référant aux dessins annexés, dans lesquels :
- la figure 1 représente l'appareillage de traitement par ionophorèse,
- la figure 2 est un schéma bloc du dispositif,
- la figure 3 est un schéma plus détaillé représentant les différents éléments constitutifs du dispositif.

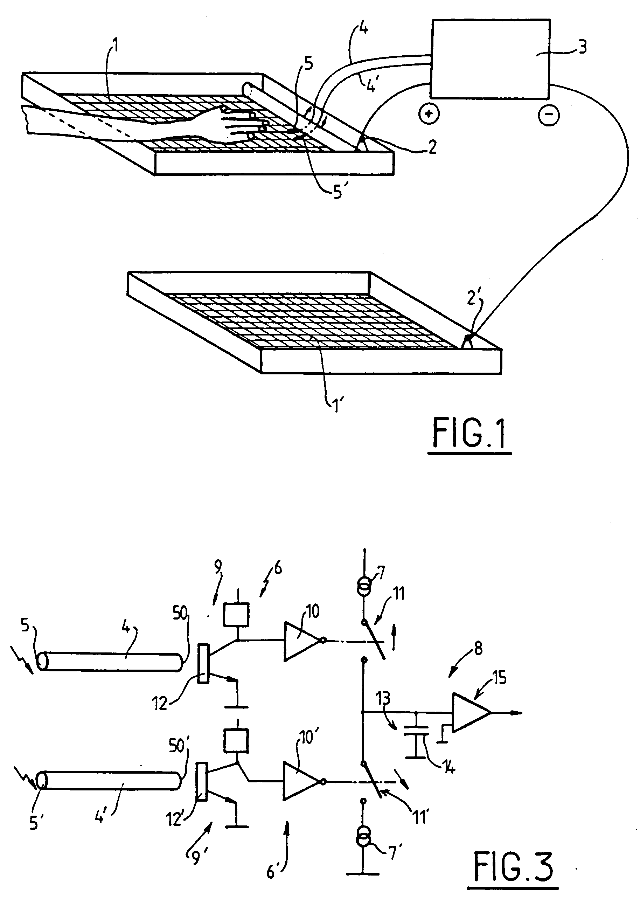

Selon la figure 1, l'appareillage de traitement par ionophorèse est constitué par deux bacs 1 et 1' remplis d'eau où l'utilisateur plonge ses mains et dans lesquels plongent respectivement deux électrodes en acier 2 et 2' respectivement reliées aux pôles positif et négatif d'un générateur de courant 3.

L'utilisateur peut régler l'intensité du courant délivré par le générateur 3 sans sortir sa main du bac 1 en obturant avec un doigt l'extrémité 5, 5' de deux fibres optiques 4, 4' qui conduisent la lumière ambiante.

L'obturation de l'extrémité 5 de la fibre 4 permet d'augmenter l'intensité du courant délivré par le générateur 3, tandis que l'obturation de l'extrémité 5' de la fibre 4' permet de diminuer cette intensité.

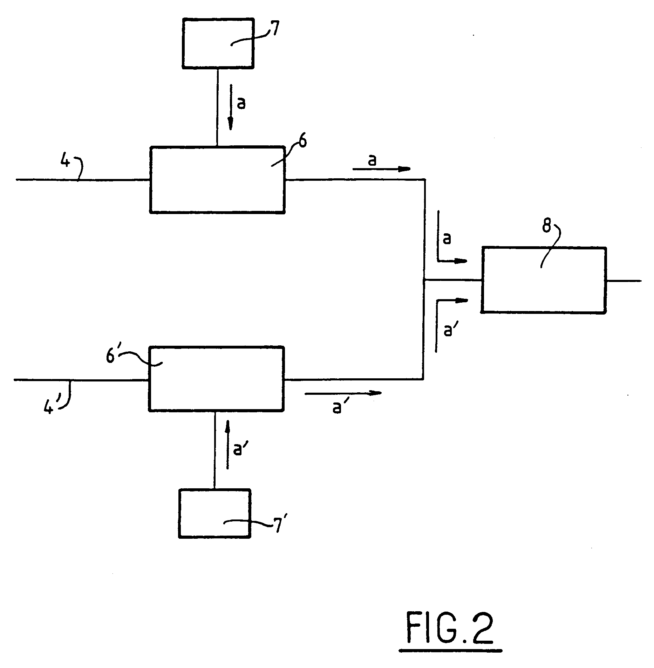

Selon la figure 2, chacune des fibres optiques 4, 4' coopère avec un commutateur opto-électronique 6, 6' qui commande la fermeture d'un circuit de réglage schématisé par les flèches a, a' renfermant un générateur de courant annexe 7, 7' ainsi qu'un organe de commande central 8 de l'intensité du courant effectivement délivré par l'appareillage représenté sur la figure 1.

Plus précisément et selon la figure 3, chacun des commutateurs opto-électroniques 6, 6' est constitué par l'association d'un phototransistor 9, 9', d'un amplificateur 10, 10' susceptible d'amplifier fortement le signal délivré par ce phototransistor 9, 9', ainsi que d'un connecteur 11, 11' dont l'actionnement par le signal susmentionné délivré par le phototransistor 9, 9' permet de commander la fermeture du circuit de réglage (flèches a et a').

La base 12, 12' du phototransistor 9, 9' coopère avec l'extrémité 50, 50' de la fibre optique 4, 4' opposée à l'extrémité 5, 5' susceptible d'être obturée par l'utilisateur de façon à permettre l'actionnement du circuit de réglage.

L'organe de commande central 8 comporte, par ailleurs, un condensateur chargé en tension 13 dont une armature 14 est reliée à la masse et qui est associé à un amplificateur opérationnel 15 ainsi qu'à un convertisseur tension/courant non représenté sur la figure.

Il est ainsi clair que le dispositif susmentionné permet le réglage du courant délivré par l'appareillage d'ionophorèse depuis un bac 1 rempli d'eau et ce, d'une façon simple, fiable et en toute sécurité.

## Revendications

1. Appareil de traitement des hyperhidroses palmoplantaires par ionophorèse constitué par deux bacs remplis d'un liquide conducteur de l'électricité, où l'utilisateur plonge ses mains et dans lesquels plongent respectivement deux électrodes en acier respectivement reliées aux pôles positif et négatif d'un générateur de courant ainsi qu'un dispositif permettant de régler l'intensité du courant délivré par le générateur de sorte qu'au début du traitement le courant soit augmenté de manière progressive et qu'à la fin de celui-ci le courant diminue également de manière progressive, appareil caractérise en ce que le dispositif permettant de régler l'intensité du courant délivré par le générateur comporte au moins une fibre optique (4, 4') conduisant la lumière ambiante et dont une extrémité ou première extrémité (5, 5') plonge dans l'un des bacs (1, 1') tandis que la seconde extrémité (50, 50') opposée à la première coopère avec des organes de réglage, l'obturation de la première extrémité (5, 5') de la ou de l'une des fibres optiques (4, 4') permettant à l'utilisateur de commander à distance l'augmentation ou la diminution du courant délivré par le générateur (3) directement avec l'une de ses mains plongeant dans le liquide de l'un des bacs (1, 1') sans avoir à sortir celle-ci.

2. Appareil selon la revendication 1, caractérisé en ce que le dispositif permettant de régler l'intensité du courant délivré par le générateur comporte au moins deux fibres optiques (4, 4'), l'obturation manuelle de l'une de ces fibres (4) ou première fibre commandant l'augmentation de l'intensité du courant délivré par le générateur (3), tandis que l'obturation de l'autre fibre (4') ou seconde fibre commande la diminution de l'intensité de ce courant.

3. Appareil selon l'une quelconque des revendications 1 et 2, caractérisé en ce que les organes de réglage comportent un commutateur opto-électronique (6, 6') respectif susceptible de commander la fermeture de circuits de réglage (a, a') respectifs renfermant un générateur de courant annexe (7, 7') ainsi qu'un organe de commande central (8).

4. Appareil selon la revendication 3, caractérisé en ce que chacun des commutateurs opto-électroniques (6, 6') comporte un élément photosensible (9, 9') dont la base (12, 12') coopère avec la seconde extrémité (50, 50') de la fibre optique (4, 4') associée, un amplificateur (10, 10') susceptible d'amplifier fortement le signal transmis par l'élément photosensible (9, 9'), ainsi qu'un connecteur (11, 11') dont l'actionnement par ce signal permet de commander la fermeture du circuit de réglage (a, a') associé.

5. Appareil selon l'une quelconque des revendications 3 et 4, caractérisé en ce que l'organe de commande central (8) comporte un condensateur (13) chargé en tension associé à un amplificateur opérationnel (15).

## Claims

1. Apparatus for the treatment of palmoplantar hyperhidroses by ionophoresis, comprising two containers filled with an electrically conductive liquid in which the user immerses his hands and in which are respectively immersed two steel electrodes which are respectively connected to the positive and negative poles of a current generator, and also a device enabling the strength of the current supplied by the generator to be adjusted in such a manner that, at the beginning of the treatment, the current is increased in a progressive manner and, at the end of the treatment, the current diminishes likewise in a progressive manner, which apparatus is characterised in that the device permitting adjustment of the strength of the current supplied by the generator comprises at least one optical fibre (4, 4') which conducts the ambient light and one end of which or the first end of which (5, 5') is immersed in one of the containers (1, 1') while the second end (50, 50') opposite the first cooperates with adjusting devices, the closure of the first end (5, 5') of the or one of the optical fibres (4, 4') enabling the user to control remotely the increase or the reduction of the current supplied by the generator (3) directly with one of his hands immersed in the liquid of one of the containers (1, 1') and without having to remove it.

2. Apparatus according to Claim 1, characterised in that the device permitting adjustment of the strength of the current supplied by the generator comprises at least two optical fibres (4, 4'), the manual closure of one of those fibres (4) or the first fibre controlling the increase in the strength of the current supplied by the generator (3), while the closure of the other fibre (4') or second fibre controls the reduction in the current strength.

3. Apparatus according to either of Claims 1 and 2, characterised in that the adjusting devices comprise a respective opto-electronic switch (6, 6') capable of controlling the closure of the respective adjustment circuits (a, a') comprising a supplementary current generator (7, 7') and also a central control unit (8).

4. Apparatus according to Claim 3, characterised in that each of the opto-electronic switches (6, 6') comprises a photosensitive element (9, 9'), the base (12, 12') of which cooperates with the second end (50, 50') of the associated optical fibre (4, 4'), an amplifier (10, 10') capable of strongly amplifying the signal transmitted by the photosensitive element (9, 9'), and also a connector (11, 11'), the actuation of which by this signal enables the closure of the associated adjustment circuit (a, a') to be controlled.

5. Apparatus according to either of Claims 3 and 4, characterised in than the central control unit (8) comprises a voltage-charged capacitor (13) associated with an operational amplifier (15).

## Patentansprüche

1. Gerät zur Behandlung von Handwurzel-Hyperhidrosen durch Ionophorese, umfassend zwei Becken, die mit einer elektrisch leitenden Flüssigkeit gefüllt sind, in welche der Benutzer seine Hände eintaucht, und in welche jeweils zwei Stahlelektroden eintauchen, die an einen positiven bzw. negativen Pol eines Stromgenerators angeschlossen sind, ferner eine Vorrichtung zum Regeln der Starke des vom Generator gelieferten Stromes, derart, daß zu Beginn der Behandlung der Strom progressiv ansteigt und während deren Ende ebenfalls progressiv abnimmt, dadurch gekennzeichnet, daß die Vorrichtung zum Regeln der Stärke des vom Generator gelieferten Stromes Wenigstens eine optische Faser (4, 4') aufweist, die Umgebungslicht leitet, und deren eines oder erstes Ende (5, 5') in eines der Becken (1, 1') eintaucht, während das zweite dem ersten abgewandte Ende (50, 50') mit einem Regler zusammenarbeitet, wobei es das Abschirmen des ersten Endes (5, 5') gegen die optische(n) Faser(n) (4, 4') dem Benutzer erlaubt, auf die Entfernung die Vergrößerung oder Verringerung des vom Generator (3) gelieferten Stromes unmittelbar mit einer seiner Hände zu steuern, die in die Flüssigkeit eines der Becken (1, 1') eintauchen, ohne diese verlassen zu müssen.

2. Gerät nach Anspruch 1, dadurch gekennzeichnet, daß die Vorrichtung, die ein Regeln der Stärke des vom Generator gelieferten Stromes erlaubt, wenigstens zwei optische Fasern (4, 4') aufweist, wobei das manuelle Abschirmen einer oder der ersten dieser Fasern (4) die Vergrößerung der Stärke des vom Generator (3) gelieferten Stromes steuert, während das Abschirmen der anderen oder zweiten Faser (4') die Verringerung der Stärke dieses Stromes steuert.

3. Gerät nach Anspruch 1 oder 2, dadurch gekennzeichnet, daß der Regler einen opto-elektronischen Wandler (6, 6') aufweist, der das Schließen der entsprechenden Steuerkreise (a, a') befiehlt, die einen zugeordneten Stromgenerator wie auch ein zentrales Steuerorgan (8) umfassen.

4. Gerät nach Anspruch 3, dadurch gekennzeichnet, daß jeder der opto-elektronischen Wandler (6, 6') ein lichtempfindliches Element (9, 9') aufweist, dessen Basis (12, 12') mit dem zweiten Ende (50, 50') der zugeordneten optischen Faser (4, 4') zusammenarbeitet, einen Verstärker (10, 10') zum starken Verstärken des von dem lichtempfindlichen Element (9, 9') übertragenen Signales, wie auch einen Schalter (11, 11'), dessen Betätigung durch das Signal das Schließen der zugehörenden Steuerschaltung (a, a') erlaubt.

5. Gerät nach Anspruch 3 oder 4, dadurch gekennzeichnet, daß das zentrale Steuerorgan (8) einen Kondensator (13) umfaßt, an den eine Spannung angelegt ist, verbunden mit einem operationalen Verstärker 15.
